# EUROPEAN PATENT SPECIFICATION

(11) **EP 4 154 807 B1**
(45) Date of publication and mention of the grant of the patent: **05.08.2026**
(21) Application number: 22195854.9
(22) Date of filing: 15.09.2022
(51) Int. Cl.: A61B 5/024, A61B 5/366, A61B 5/00, A61N 1/362, A61N 1/37

(54) **PHYSIOLOGICAL INFORMATION PROCESSING APPARATUS, PHYSIOLOGICAL INFORMATION PROCESSING METHOD AND PROGRAM**
VORRICHTUNG ZUR VERARBEITUNG PHYSIOLOGISCHER INFORMATIONEN, VERFAHREN ZUR VERARBEITUNG PHYSIOLOGISCHER INFORMATIONEN UND PROGRAMM
APPAREIL DE TRAITEMENT D'INFORMATIONS PHYSIOLOGIQUES, PROCÉDÉ ET PROGRAMME DE TRAITEMENT D'INFORMATIONS PHYSIOLOGIQUES

(30) Priority: 22.09.2021 JP 2021154160
(43) Date of publication of application: 29.03.2023
(73) Proprietor: Nihon Kohden Corporation, Shinjuku-ku Tokyo (JP)
(72) Inventor: Kimura, Kei, Saitama, 3590037 (JP)
(74) Representative: Grünecker Patent- und Rechtsanwälte PartG mbB

(56) References cited:
- EP-A2- 0 424 607
- US-A1- 2004 116 969
- VESSELA KRASTEVA ET AL: "Real-time arrhythmia detection with supplementary ECG quality and pulse wave monitoring for the reduction of false alarms in ICUs", PHYSIOLOGICAL MEASUREMENT, INSTITUTE OF PHYSICS PUBLISHING, BRISTOL, GB, vol. 37, no. 8, 25 July 2016 (2016-07-25), pages 1273 - 1297, XP020307063, ISSN: 0967-3334, [retrieved on 20160725], DOI: 10.1088/0967-3334/37/8/1273

## Description

### TECHNICAL FIELD

The presently disclosed subject matter relates to a physiological information processing apparatus and a physiological information processing method. The presently disclosed subject matter further relates to a program that causes a computer to execute the physiological information processing method.

### BACKGROUND ART

When an electrocardiogram waveform of a patient with a cardiovascular implantable electronic device (CIED) such as a pacemaker is measured, a pacing pulse output from the electronic device may be erroneously recognized as a QRS complex by a patient monitor. As a result, reliability of a diagnosis result of a cardiac function of the patient based on electrocardiogram data decreases.

On the other hand, in consideration of a fact that the pacing pulse affects the QRS complex, Patent Literature 1 discloses a processing method for preventing a patient monitor from detecting a QRS complex present near the time of a detected pacing pulse.

### CITATION LIST

### PATENT LITERATURE

Patent Literature 1: Japanese Patent No. 2635079
EP 0 424 607 A2 provides a method for discriminating pace pulse tails. A method is described by which a paced ECG signal can be analyzed to discriminate pace pulse tails from QRS complexes.

Vessela Krasteva at al.: "Real-time arrhythmia detection with supplementary ECG quality and pulse wave monitoring for the reduction of false alarms in ICUs", Physiological Measurement, Institute of physics publishing, Bristol, GB, Vol. 37, no. 8, 25 July 2016, pages 1273 - 1297, ISSN: 0967-3334, DOI: 10.1088/0967-3334/37/8/1273 mentions a QRS detection module where pre-filters based on a non-linear filtering approach that completely reset deviations from the neighboring preceding value are embedded to prevent the QRS detector from erroneously triggering on pacemaker spikes.

US 2004/0116969 A1 deals with pulse detection using patient physiological signals. Detection of QRS complexes in the patient's ECG may be used as patient physiological signal data to identify pacing capture.

When no QRS complex present near the time of pacing pulse is detected, a healthcare worker cannot accurately grasp an actual state of a cardiac function of a patient with a pacemaker or the like. In this regard, for example, even when the cardiac function of the patient is in a normal state, a time interval between adjacent QRS complexes becomes large when none of a plurality of QRS complexes is detected due to the presence of pacing pulses. As a result, it is assumed that an alarm indicating an abnormality of the cardiac function of the patient is erroneously output from the patient monitor. In this way, from the above viewpoint, there is room for consideration of a processing apparatus that can suitably prevent a situation in which the actually present QRS complex is not detected due to the pacing pulse.

### SUMMARY

The invention provides a physiological information processing apparatus according to claim 1 and a physiological information processing method according to claim 1.

An object of the presently disclosed subject matter is to provide a physiological information processing apparatus and a physiological information processing method that can suitably prevent a situation in which an actually present QRS complex is not detected due to a pacing pulse.

A physiological information processing apparatus according to a first aspect of the presently disclosed subject matter includes:
one or more processors; and
one or more memories configured to store a computer readable command.

In a case where the computer readable command is executed by the processor, the physiological information processing apparatus:
acquires electrocardiogram data of a subject;
detects a QRS complex from the electrocardiogram data;
acquires pulse wave data of the subject;
detects a pacing pulse output from an apparatus worn by the subject;
determines whether the pacing pulse is detected in a time period in which the QRS complex is not detected when the time period is equal to or longer than a predetermined time width;
determines, based on the pulse wave data, whether a pulse wave is present within predetermined time from a detection time point at which the pacing pulse is detected; and
determines, according to a determination that the pulse wave is present within the predetermined time, that the QRS complex is actually present in the time period.

According to the above configuration, the physiological information processing apparatus determines whether the pacing pulse is present in the time period in which the QRS complex is not detected when the time period is equal to or longer than the predetermined time width. Further, the physiological information processing apparatus determines, according to the determination that the pulse wave related to the QRS complex is present within the predetermined time from the detection time point at which the pacing pulse is detected, that the QRS complex is actually present in the time period. In this way, it is possible to provide a physiological information processing apparatus that can suitably prevent a situation in which an actually present QRS complex is not detected due to a pacing pulse.

A physiological information processing method executed by a computer according to a second aspect of the presently disclosed subject matter includes:
acquiring electrocardiogram data of a subject;
detecting a QRS complex from the electrocardiogram data;
acquiring pulse wave data of the subject;
detecting a pacing pulse output from an apparatus implanted in a body of the subject;
determining whether the pacing pulse is detected in a time period in which the QRS complex is not detected when the time period is equal to or longer than a predetermined time width;
determining, based on the pulse wave data, whether a pulse wave is present within predetermined time from a detection time point at which the pacing pulse is detected; and
determining, according to a determination that the pulse wave is present within the predetermined time, that the QRS complex is actually present in the time period.

There is provided a program that causes a computer to execute the physiological information processing method.

According to the presently disclosed subject matter, it is possible to provide a physiological information processing apparatus and a physiological information processing method that can suitably prevent a situation in which an actually present QRS complex is not detected due to a pacing pulse.

### BRIEF DESCRIPTION OF DRAWINGS

FIG. 1 is a hardware configuration diagram illustrating a physiological information processing apparatus according to the presently disclosed subject matter (hereinafter, simply referred to as the present embodiment).
FIG. 2 is a diagram illustrating an example of a configuration of a sensor interface.
FIG. 3 is a flowchart illustrating a process of obtaining a feature related to a QRS complex from electrocardiogram data.
FIG. 4 is a flowchart illustrating a process of obtaining a feature related to a pulse wave from pulse wave data.
FIG. 5 is a flowchart illustrating a physiological information processing method according to the present embodiment.
FIG. 6 is a diagram illustrating a QRS complex not detected due to a pacing pulse and a pulse wave present within predetermined time from a detection time point of the pacing pulse.

### DESCRIPTION OF EMBODIMENTS

Hereinafter, an embodiment will be described with reference to the drawings. For convenience of description, description of elements having the same reference numerals as those already described in the description of the present embodiment will be omitted.

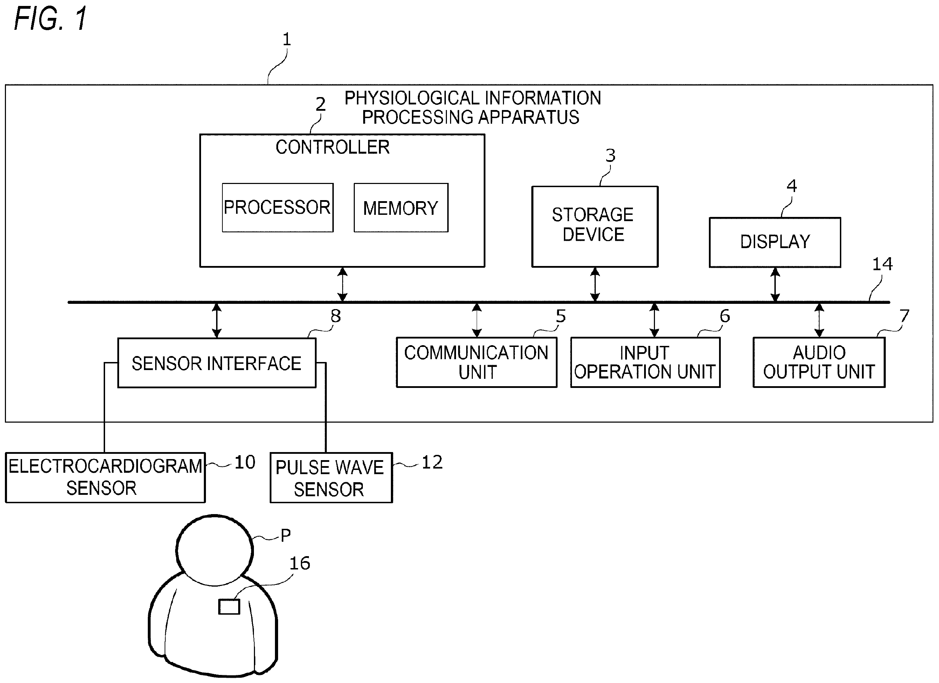

FIG. 1 illustrates a hardware configuration diagram of a physiological information processing apparatus 1 according to the present embodiment. As illustrated in FIG. 1, the physiological information processing apparatus 1 (hereinafter, simply referred to as the processing apparatus 1) includes a controller 2, a storage device 3, a display 4, a communication unit 5, an input operation unit 6, an audio output unit 7, and a sensor interface 8. These components provided in the processing apparatus 1 are communicably connected to each other via a bus 14.

The processing apparatus 1 may be a medical instrument (for example, a patient monitor) that displays physiological information on a subject P, a personal computer, a workstation, a smartphone, a tablet, or a wearable device (for example, an AR glass) worn on a body (for example, an arm, a head) of a healthcare worker.

The subject P wears a pacemaker 16 that is an example of a CIED. The pacemaker 16 is implanted in the body of the subject P, and outputs a pacing pulse that applies electrical stimulation to the heart of the subject P. The timing at which the pacing pulse is output from the pacemaker 16 may overlap the output timing of a QRS complex in an electrocardiogram waveform indicating an electrical activity of the heart of the subject P. The QRS complex on which the pacing pulse is superimposed is generally referred to as a fusion beat or a pseudofusion beat. In this case, the pacing pulse adversely affects the QRS complex, and accordingly reliability of a diagnosis result of a cardiac function of the subject P based on a feature related to the QRS complex may decrease. In this way, the processing apparatus 1 according to the present embodiment does not store data related to the QRS complex present near the pacing pulse on a timeline when the output timing of the pacing pulse substantially overlaps the output timing of the QRS complex. That is, although the QRS complex present near the pacing pulse on the timeline is actually present on electrocardiogram data, the processing apparatus 1 does not detect the QRS complex or store data related to the QRS complex. On the other hand, the processing apparatus 1 according to the present embodiment has a function of determining, based on pulse wave data, whether the QRS complex not detected due to the presence of the pacing pulse is actually present. Details of the determination function will be described later.

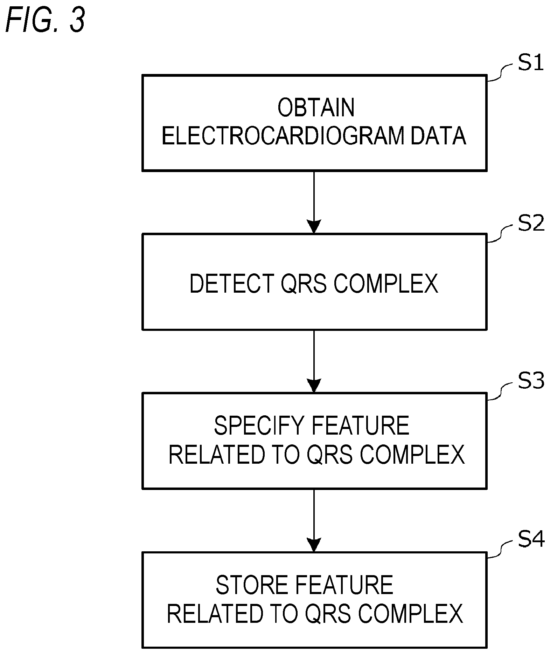

The controller 2 includes one or more memories and one or more procesors. The memory stores a computer readable command (program). The memory includes, for example, a read only memory (ROM) that stores various programs and the like, and a random access memory (RAM) having a plurality of work areas in which various programs and the like to be executed by the processor are stored. The processor includes, for example, at least one of a central processing unit (CPU), a micro-processing unit (MPU), and a graphics processing unit (GPU). The CPU may include a plurality of CPU cores. The GPU may include a plurality of GPU cores. The processor may load a designated program from various programs provided in the storage device 3 or the ROM onto the RAM and execute various processes in cooperation with the RAM. In particular, when the processor loads a physiological information processing program for executing a series of processes illustrated in FIGS. 3 to 5 onto the RAM and executes the program in cooperation with the RAM, the controller 2 executes the series of processes illustrated in FIGS. 3 to 5. Details of the physiological information processing program will be described later.

The storage device 3 is, for example, a storage device (storage) such as a hard disk drive (HDD), a solid state drive (SSD), or a flash memory, and stores programs and various data. The physiological information processing program may be provided in the storage device 3. Further, physiological information data (electrocardiogram data, pulse wave data, or the like) indicating physiological information on the subject P may be stored in the storage device 3. For example, electrocardiogram data obtained by an electrocardiogram sensor 10 may be stored in the storage device 3 via the sensor interface 8.

The communication unit 5 connects the processing apparatus 1 to an in-hospital network. Specifically, the communication unit 5 may include various wired connection terminals that communicate with a central monitor or a server provided in the in-hospital network. The communication unit 5 may further include a wireless communication module that performs wireless communication with the central monitor or the server. The communication unit 5 may include, for example, a wireless communication module corresponding to a medical telemeter system. The communication unit 5 may include a wireless communication module corresponding to a wireless communication standard such as Wi-Fi (registered trademark) or Bluetooth (registered trademark) and/or a wireless communication module corresponding to a mobile communication system using a SIM. The in-hospital network may be, for example, a local area network (LAN) or a wide area network (WAN). The processing apparatus 1 may be connected to the Internet via the in-hospital network.

The display 4 displays information (for example, an electrocardiogram waveform or a pulse wave) related to physiological information data of the subject P obtained in real time, and is, for example, a liquid crystal panel or an organic EL panel. Further, the display 4 displays, on a display screen, an alarm in accordance with a time interval between adjacent QRS complexes on a timeline. The input operation unit 6 is, for example, a touch panel overlapping the display 4, a mouse, and/or a keyboard. The input operation unit 6 receives an input operation by the healthcare worker and generates an operation signal corresponding to the input operation by the healthcare worker. After the operation signal generated by the input operation unit 6 is transmitted to the controller 2 via the bus 14, the controller 2 executes a predetermined operation in accordance with the operation signal. The audio output unit 7 includes one or more speakers, and outputs an audio alarm in accordance with the time interval between adjacent QRS complexes on the timeline.

The sensor interface 8 is an interface that connects a vital sensor such as the electrocardiogram sensor 10 and a pulse wave sensor 12 to the processing apparatus 1. The sensor interface 8 may include an input terminal to which a physiological signal output from a vital sensor is input. The input terminal may be physically connected to a connector of the vital sensor. The electrocardiogram sensor 10 obtains an electrocardiogram signal indicating an electrical activity of the heart of the subject P, and includes a plurality of ECG electrodes attached to the subject P. The processing apparatus 1 generates electrocardiogram data based on an electrocardiogram signal output from the electrocardiogram sensor 10 and detects a pacing pulse. The pulse wave sensor 12 obtains a pulse wave signal indicating a pulse wave of the subject P. The pulse wave sensor 12 may include, for example, a light emitter (for example, an LED) that emits red light and/or infrared light, and a light detecting element (for example, a photodiode) that detects light that is emitted from the light emitter and that passes through the finger of the subject P. The processing apparatus 1 generates pulse wave data based on a pulse wave signal output from the pulse wave sensor 12. Further, the processing apparatus 1 may obtain information on arterial transcutaneous oxygen saturation (SpO2) and/or blood pressure of the subject P based on the generated pulse wave data.

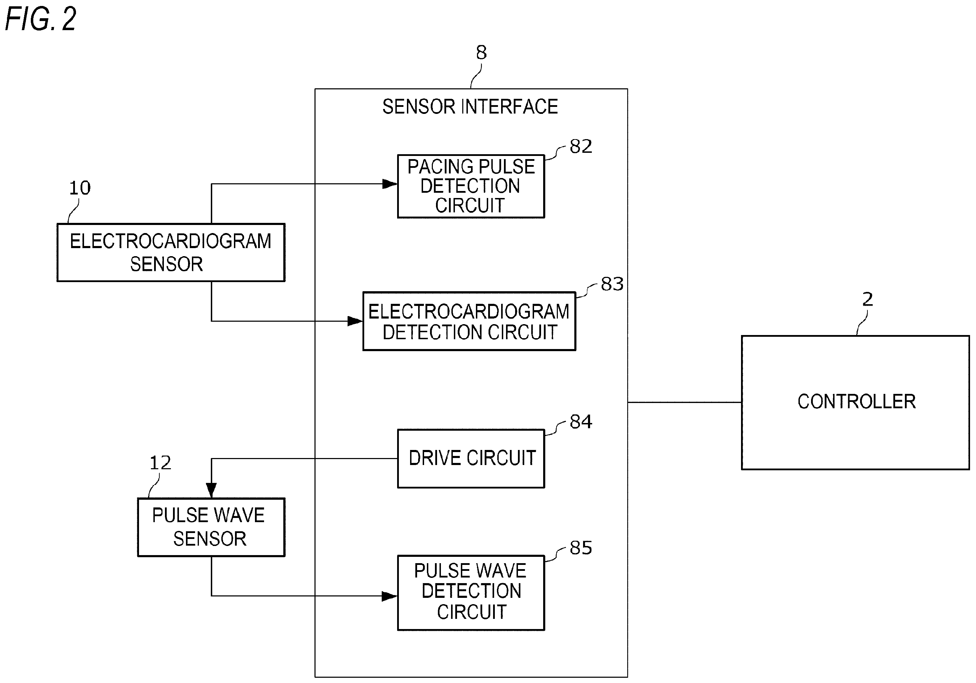

Next, the sensor interface 8 will be described in detail with reference to FIG. 2. As illustrated in FIG. 2, the sensor interface 8 includes a pacing pulse detection circuit 82, an electrocardiogram detection circuit 83, a drive circuit 84, and a pulse wave detection circuit 85.

The pacing pulse detection circuit 82 detects a pacing pulse based on a highfrequency component (frequency band of several kHz) of the electrocardiogram signal output from the electrocardiogram sensor 10. The pacing pulse detection circuit 82 includes, for example, at least a high-pass filter and a comparator. The electrocardiogram detection circuit 83 generates electrocardiogram data (digital data) based on a low-frequency component (frequency band of several Hz) of the electrocardiogram signal output from the electrocardiogram sensor 10. The electrocardiogram detection circuit 83 includes at least a differential amplifier circuit, a low-pass filter, and an AD converter. The drive circuit 84 generates a drive signal for driving the pulse wave sensor 12 and then supplies the drive signal to the pulse wave sensor 12. The pulse wave detection circuit 85 generates pulse wave data (digital data) based on the pulse wave signal output from the pulse wave sensor 12. The pulse wave detection circuit 85 includes at least an amplifier circuit, a filter circuit, and an AD converter.

In this way, the sensor interface 8 generates the electrocardiogram data (digital data) based on the electrocardiogram signal (analog signal) output from the electrocardiogram sensor 10, and then transmits the electrocardiogram data to the controller 2. The sensor interface 8 generates the pulse wave data (digital data) based on the pulse wave signal (analog signal) output from the pulse wave sensor 12, and then transmits the pulse wave data to the controller 2.

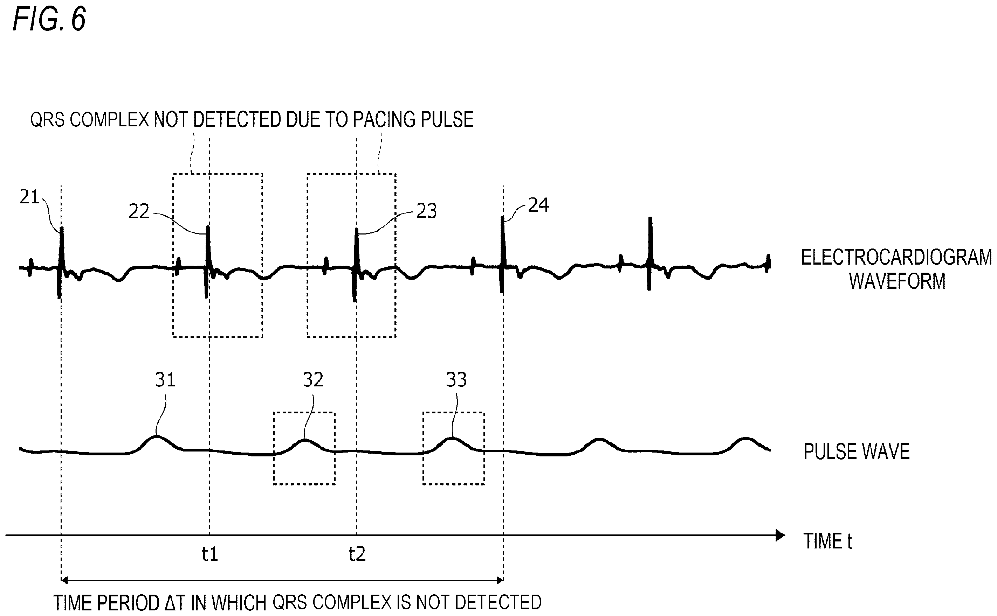

Next, a process of obtaining a feature related to the QRS complex based on electrocardiogram data will be described below with reference to FIG. 3. FIG. 3 is a flowchart illustrating a process of obtaining a feature related to the QRS complex based on electrocardiogram data. As illustrated in FIG. 3, in step S1, the controller 2 obtains electrocardiogram data from the electrocardiogram sensor 10 via the sensor interface 8. In step S2, the controller 2 detects a QRS complex indicating excitation of the ventricle from the electrocardiogram data based on a predetermined detection algorithm. For example, an example of a plurality of QRS complexes is illustrated in an upper part of FIG. 6.

Next, the controller 2 specifies a feature related to the QRS complex for each of the plurality of QRS complexes (step S3). The feature related to the QRS complex includes, for example, at least one of an interval between adjacent QRS complexes on a timeline (specifically, an RR interval indicating an interval between adjacent R waves), a time width of the QRS complex, an amplitude of the QRS complex, a maximum value/minimum value of the QRS complex, and an area of the QRS complex. Thereafter, the controller 2 stores the feature related to the QRS complex in the memory or the storage device 3 (step S4). In this case, the controller 2 may store a moving average value (for example, a moving average value of 16 beats) of the feature related to the QRS complex.

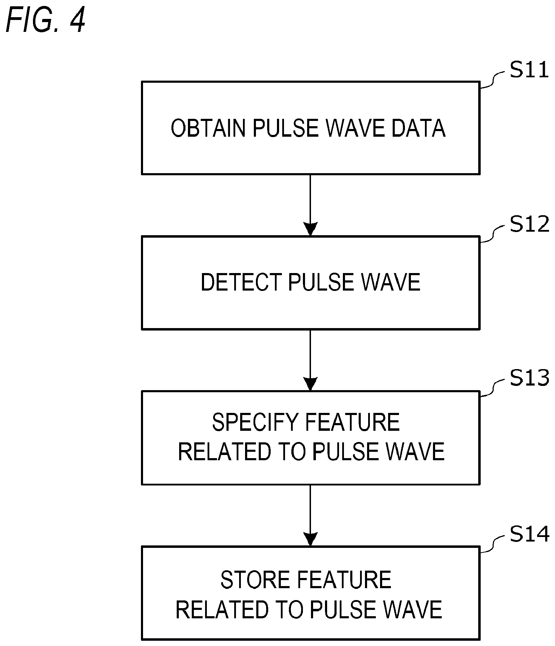

Further, a process of obtaining a feature related to a pulse wave based on pulse wave data will be described below with reference to FIG. 4. FIG. 4 is a flowchart illustrating a process of obtaining a feature related to a pulse wave based on pulse wave data. As illustrated in FIG. 4, in step S11, the controller 2 obtains pulse wave data from the pulse wave sensor 12 via the sensor interface 8. In step S12, the controller 2 detects a pulse wave from the pulse wave data based on a predetermined detection algorithm. For example, an example of a plurality of pulse waves is illustrated in a lower part of FIG. 6.

Next, the controller 2 specifies a feature related to the pulse wave for each of the plurality of pulse waves (step S13). The feature related to the pulse wave includes, for example, at least one of an amplitude of the pulse wave, a time width of the pulse wave, a maximum value/minimum value of the pulse wave, contraction time of the pulse wave, and delay time of the pulse wave. The "contraction time of the pulse wave" is time from a time point corresponding to a rising point of the pulse wave to a time point corresponding to a peak point of the pulse wave. The "delay time of the pulse wave" corresponds to a time interval between a detection time point at which the pulse wave is detected and a detection time point at which the QRS complex associated with the pulse wave (particularly, the QRS complex appearing immediately before the pulse wave) is detected. More specifically, the "delay time of the pulse wave" may be a time interval between the time point corresponding to the rising point of the pulse wave and a time point corresponding to a rising point of the QRS complex appearing immediately before the pulse wave, or a time interval between the time point corresponding to the peak point of the pulse wave and a time point corresponding to the peak point of the QRS complex appearing immediately before the pulse wave. Thereafter, the controller 2 stores the feature related to the pulse wave in the memory or the storage device 3 (step S14). In this case, the controller 2 may store a moving average value (for example, a moving average value of 16 beats) of the feature related to the pulse wave.

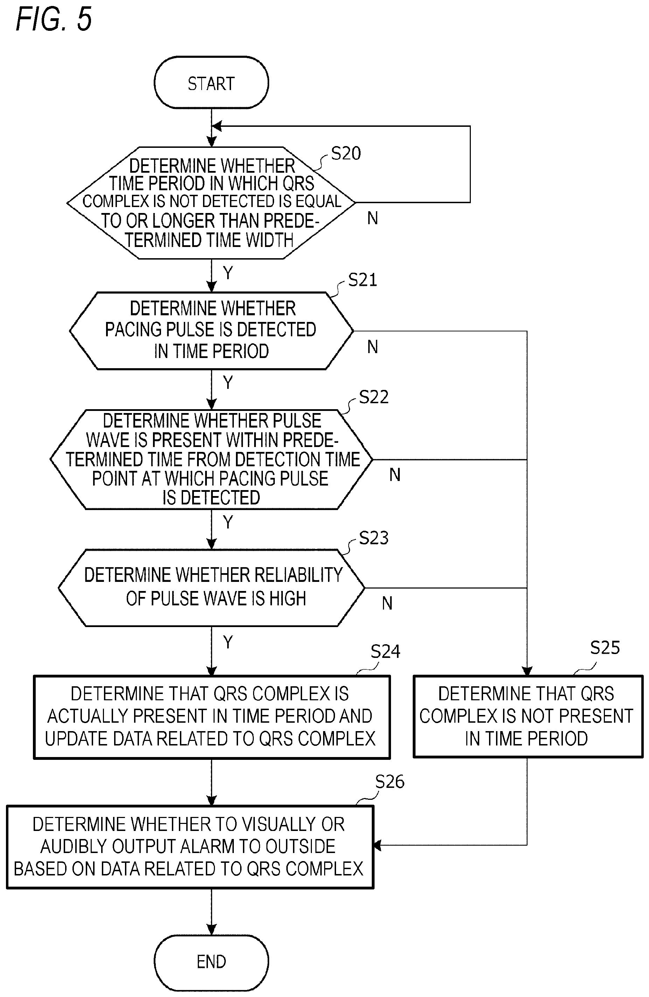

Next, a physiological information processing method according to the present embodiment will be described below with reference to FIGS. 5 and 6. FIG. 5 is a flowchart illustrating the physiological information processing method according to the present embodiment. FIG. 6 is a diagram illustrating QRS complexes 22, 23 not detected due to the pacing pulse and pulse waves 32, 33 present within predetermined time from detection time points t1, t2 of the pacing pulse. As a precondition, the processing apparatus 1 according to the present embodiment does not detect the QRS complex present near the pacing pulse on a timeline. It is further assumed that the processing apparatus 1 executes an example of the process illustrated in FIG. 5 while executing the series of processes illustrated in FIG. 3 (that is, while obtaining the QRS complex from the electrocardiogram data).

As illustrated in FIG. 5, in step S20, the controller 2 determines whether a time period ΔT in which the QRS complex is not detected is equal to or longer than a predetermined time width. Here, the predetermined time width is, for example, a predetermined value between 200 milliseconds and 2 seconds. When the controller 2 detects that the time period ΔT is equal to or longer than the predetermined time width (Yes in step S20), the controller 2 executes the processing of step S21. On the other hand, when the determination processing of step S20 is No, the controller 2 waits until the time period ΔT is equal to or longer than the predetermined time width.

In the example illustrated in FIG. 6, since the QRS complexes 22, 23 overlap the pacing pulse on the timeline, the QRS complexes 22, 23 are not detected by the processing apparatus 1. That is, the time period ΔT in which the QRS complex is not detected is a time period from a time point corresponding to a peak point of a QRS complex 21 to a time point corresponding to a peak point of a QRS complex 24. In addition, it is assumed that the pacing pulse is detected at the time points t1, t2.

Next, in step S21, the controller 2 determines whether a pacing pulse is detected in the time period ΔT. In particular, the controller 2 determines whether a pacing pulse is detected in the time period ΔT based on information output from the pacing pulse detection circuit 82. When a determination result of step S21 is Yes, the process proceeds to step S22. On the other hand, when the determination result of step S21 is No, the process proceeds to step S25. In this case, the controller 2 determines that the QRS complex is not present in the time period ΔT (step S25), and then executes the processing of step S26.

In step S22, the controller 2 determines, based on pulse wave data, whether a pulse wave is present within predetermined time from a detection time point at which a pacing pulse is detected. Here, the predetermined time is, for example, 1 second. In the example illustrated in FIG. 6, the controller 2 determines whether the pulse wave 32 is present within predetermined time from the detection time point t1, and determines whether the pulse wave 33 is present within predetermined time from the detection time point t2, based on the pulse wave data. When a determination result of step S22 is Yes, the process proceeds to step S23, and when the determination result of step S22 is No, the process proceeds to step S25.

In step S23, the controller 2 determines whether reliability of the pulse wave present within the predetermined time is high. In particular, the controller 2 may determine the reliability of the pulse wave by comparing a feature of the pulse wave present within the predetermined time with a feature of a pulse wave stored in the memory. As described above, the feature of the pulse wave includes at least one of the amplitude of the pulse wave, the time width of the pulse wave, the maximum value/minimum value of the pulse wave, the contraction time of the pulse wave, and the delay time of the pulse wave.

For example, the controller 2 may determine that the reliability of the pulse wave 32 is high when a feature of the pulse wave 32 present within the predetermined time satisfies a part or all of the following conditions.
·The amplitude of the pulse wave 32 is 0.25 to less than 3 times an average amplitude of the pulse wave stored in the memory.
·The delay time of the pulse wave 32 from the detection time point t1 of the pacing pulse to the detection time point of the pulse wave 32 is within ± 80 ms of average delay time of the pulse wave stored in the memory.
·The delay time of the pulse wave 32 from the detection time point t1 of the pacing pulse to the detection time point of the pulse wave 32 is within ± 80 ms of the delay time of the pulse wave 31 adjacent to the pulse wave 32.
·The contraction time of the pulse wave 32 is not longer than average contraction time of the pulse wave stored in the memory by 120 ms or more.

The controller 2 may determine the reliability of the pulse wave by determining whether noise is superimposed on the pulse wave present within the predetermined time, in addition to the comparison between the feature of the pulse wave present within the predetermined time and the feature of the pulse wave stored in the memory. For example, when the reliability of the pulse wave 32 is high in a comparison result between the feature of the pulse wave 32 and the feature of the pulse wave stored in the memory and noise is superimposed on the pulse wave, the controller 2 may determine that the reliability of the pulse wave 32 is not high. When the reliability of the pulse wave 32 is high in the comparison result between the feature of the pulse wave 32 and the feature of the pulse wave stored in the memory and noise is not superimposed on the pulse wave 32, the controller 2 may determine that the reliability of the pulse wave 32 is high. In this way, the reliability of the pulse wave is determined in consideration of the noise superimposed on the pulse wave, and thus it is possible to more accurately determine the reliability of the pulse wave.

When a determination result of step S23 is Yes, the process proceeds to step S24, and when the determination result of step S23 is No, the process proceeds to step S25. Next, in step S24, the controller 2 determines that the QRS complex is actually present in the time period ΔT, and updates data related to the QRS complex. In the example illustrated in FIG. 6, the controller 2 can determine that the two QRS complexes 22, 23 are actually present within the time period ΔT after determining that the reliability of the two pulse waves 32, 33 is high.

Further, the controller 2 may update, as the data related to the QRS complex, information related to the time interval between adjacent QRS complexes (in particular, information related to the RR interval). In this regard, the controller 2 may store the detection time point t1 at which the pacing pulse is detected as a detection time point at which the QRS complex 22 is detected (in particular, the time point corresponding to the peak point of the QRS complex 22), and may store the detection time point t2 at which the pacing pulse is detected as a detection time point at which the QRS complex 23 is detected (in particular, the time point corresponding to the peak point of the QRS complex 23).

Next, in step S26, the controller 2 determines whether to visually or audibly output an alarm indicating an abnormality of the cardiac function of the subject P to the outside based on the data related to the QRS complex. In particular, the controller 2 may determine whether to output an alarm to the outside in accordance with a comparison between the time interval between adjacent QRS complexes and a predetermined threshold value Tth. Here, the predetermined threshold value Tth may be set within a range of 3 seconds to 5 seconds, or be a value N times (N > 1) the average value of the measured RR interval. For example, when the time interval (RR interval) between adjacent QRS complexes is equal to or greater than the predetermined threshold value Tth, the controller 2 may determine to output an alarm to the outside and then output the alarm to the outside through the audio output unit 7 and/or the display 4. On the other hand, when the time interval between the adjacent QRS complexes is smaller than the predetermined threshold value Tth, the controller 2 determines not to output the alarm to the outside.

The controller 2 may output a plurality of types of alarms to the outside according to the state of the cardiac function of the subject P (severity level of the cardiac function). In this case, the controller 2 may change the type of an alarm to be output to the outside according to the time interval between adjacent QRS complexes. For example, the controller 2 may output a first alarm to the outside when the time interval is between a first threshold value Tth1 and a second threshold value Tth2. When the time interval is larger than the second threshold value Tth2, the controller 2 may output a second alarm different from the first alarm to the outside.

In the example illustrated in FIG. 6, since it is determined that the QRS complexes 22, 23 not detected due to the pacing pulse are actually present, the information on the time interval between adjacent QRS complexes is updated. As a result, since the time interval between adjacent QRS complexes does not exceed the predetermined threshold value Tth, no alarm is output from the processing apparatus 1. In this way, when the cardiac function of the subject P is normal, it is possible to suitably prevent a situation in which an alarm indicating an abnormality of the cardiac function of the subject P is erroneously output from the processing apparatus 1. On the other hand, in a processing apparatus in the related art, since it is not determined whether the QRS complexes 22, 23 not detected by the pacing pulse are actually present, the time interval between adjacent QRS complexes exceeds the predetermined threshold value Tth. In this example, since the time interval ΔT between the time point corresponding to the peak point of the QRS complex 21 and the time point corresponding to the peak point of the QRS complex 24 exceeds the predetermined threshold value Tth, an alarm is output from the processing apparatus. As a result, although the cardiac function of the subject P is normal, an alarm indicating an abnormality of the cardiac function of the subject P is erroneously output from the processing apparatus.

As described above, according to the present embodiment, it is possible to suitably prevent a situation in which the actually present QRS complex is not detected due to the pacing pulse. Furthermore, when the cardiac function of the subject P wearing the pacemaker 16 is normal, it is possible to suitably prevent a situation in which an alarm indicating an abnormality of the cardiac function of the subject P is erroneously output. Therefore, it is possible to provide the processing apparatus 1 that can more accurately determine the state of the cardiac function of the subject P wearing the pacemaker.

According to the present embodiment, it is determined that the QRS complex is actually present in the time period ΔT according to the determination of a high reliability of the pulse wave present within predetermined time (for example, within 1 second) from the detection time point of the pacing pulse. In this way, it is possible to more reliably determine that the QRS complex is actually present within the time period ΔT, and it is possible to more accurately determine the state of the cardiac function of the subject P.

In the description of the present embodiment, the determination processing (step S23) related to the reliability of the pulse wave is executed, but the determination processing of step S23 is not necessarily executed. In this case, after determining in step S22 that the pulse wave is present within predetermined time from the detection time point at which the pacing pulse is detected, the controller 2 may determine that the QRS complex is actually present in the time period ΔT and update the data related to the QRS complex.

In order to implement the processing apparatus 1 according to the present embodiment by software, a physiological information processing program may be pre-loaded into the storage device 3 or the ROM. Alternatively, the physiological information processing program may be stored in a computer-readable storage medium such as a magnetic disk (for example, a HDD or a floppy disk), an optical disk (for example, a CD-ROM, a DVD-ROM, or a Blu-ray (registered trademark) disk), a magneto-optical disk (for example, a MO), or a flash memory (for example, a SD card, a USB memory, or a SSD). In this case, the physiological information processing program stored in the storage medium may be loaded into the storage device 3. Further, the program assembled in the storage device 3 may be loaded onto the RAM, and then the processor may execute the program loaded onto the RAM. In this way, the physiological information processing method according to the present embodiment is executed by the processing apparatus 1.

The physiological information processing program may be downloaded from a computer on a communication network via the communication unit 5. In this case, same or similarly, the downloaded program may be loaded into the storage device 3.

Although the embodiment of the presently disclosed subject matter is described above, the technical scope of the presently disclosed subject matter should not be construed as being limited to the description of the embodiment. It is understood by those skilled in the art that the present embodiment is an example and various modifications can be made within the scope of the inventions described in the claims. The technical scope of the presently disclosed subject matter should be determined based on the scope of the inventions described in the claims.

## Claims

1. A physiological information processing apparatus comprising:
one or more processors; and
one or more memories configured to store a computer readable command,
wherein in a case where the computer readable command is executed by the processor, the physiological information processing apparatus is configured to:
acquire (S1) electrocardiogram data of a subject;
detect (S2) a QRS complex from the electrocardiogram data;
acquire (S11) pulse wave data of the subject;
detect (S21) a pacing pulse output from an apparatus worn by the subject;
determine (S20, S21) whether the pacing pulse is detected in a time period in which a QRS complex is not detected when the time period is equal to or longer than a predetermined time width and if the QRS complex has not been detected due to the presence of the pacing pulse,
determine (S22), based on the pulse wave data, whether a pulse wave is present within predetermined time from a detection time point at which the pacing pulse is detected; and
determine (S24), according to a determination that the pulse wave is present within the predetermined time, that the QRS complex is actually present in the time period.

2. The physiological information processing apparatus according to claim 1,
wherein the physiological information processing apparatus is configured to:
update data related to the QRS complex according to the determination that the pulse wave is present within the predetermined time; and
determine whether to audibly or visually output an alarm indicating an abnormality of a cardiac function of the subject to outside based on the data related to the QRS complex.

3. The physiological information processing apparatus according to claim 2,
wherein the data related to the QRS complex includes information related to a time interval between adjacent QRS complexes on a timeline, and
wherein the physiological information processing apparatus is configured to determine whether to audibly or visually output the alarm to the outside in accordance with a comparison between the time interval and a predetermined threshold value.

4. The physiological information processing apparatus according to any one of claims 1 to 3, wherein the physiological information processing apparatus is configured to:
determine (S23) reliability of the pulse wave when the pulse wave is determined to be present within the predetermined time; and
determine that the QRS complex is actually present in the time period according to a determination that the reliability of the pulse wave is high.

5. The physiological information processing apparatus according to claim 4,
wherein the physiological information processing apparatus is configured to determine the reliability of the pulse wave by comparing a feature of the pulse wave present within the predetermined time with a feature of a pulse wave stored in the memory.

6. The physiological information processing apparatus according to claim 5,
wherein the physiological information processing apparatus is configured to determine the reliability of the pulse wave by comparing the feature of the pulse wave present within the predetermined time with the feature of the pulse wave stored in the memory, and by determining whether noise is superimposed on the pulse wave present within the predetermined time.

7. The physiological information processing apparatus according to claim 5 or 6, wherein the feature of the pulse wave includes at least one of:
a feature related to an amplitude of the pulse wave;
a feature related to contraction time of the pulse wave; and
a feature related to delay time of the pulse wave corresponding to a time interval between a detection time point at which the pulse wave is detected and a detection time point at which the QRS complex associated with the pulse wave or the pacing pulse is detected.

8. The physiological information processing apparatus according to claim 2 or 3,
wherein the physiological information processing apparatus is configured to update, according to the determination that the pulse wave is present within the predetermined time, the data related to the QRS complex by storing a detection time point at which the pacing pulse is detected as a detection time point at which the QRS complex is detected.

9. A physiological information processing method executed by a computer, the method comprising:
Acquiring (S1) electrocardiogram data of a subject;
Detecting (S2) a QRS complex from the electrocardiogram data;
Acquiring (S11) pulse wave data of the subject;
Detecting (S21) a pacing pulse output from an apparatus implanted in a body of the subject;
Determining (S20, S21) whether the pacing pulse is detected in a time period in which a QRS complex is not detected when the time period is equal to or longer than a predetermined time width;
If the QRS complex has not been detected due to the presence of the pacing pulse, determining (S22), based on the pulse wave data, whether a pulse wave is present within predetermined time from a detection time point at which the pacing pulse is detected; and
Determining (S24), according to a determination that the pulse wave is present within the predetermined time, that the QRS complex is actually present in the time period.

10. The physiological information processing method according to claim 9, further comprising:
updating data related to the QRS complex according to the determination that the pulse wave is present within the predetermined time; and
determining whether to audibly or visually output an alarm to outside based on the data related to the QRS complex.

11. The physiological information processing method according to claim 10,
wherein the data related to the QRS complex includes information related to a time interval between adjacent QRS complexes on a timeline, and
wherein the method further includes determining whether to audibly or visually output the alarm to the outside in accordance with a comparison between the time interval and a predetermined threshold value.

12. The physiological information processing method according to any one of claims 9 to 11, further comprising:
Determining (S23) reliability of the pulse wave when the pulse wave is determined to be present within the predetermined time, and
wherein the method further includes determining that the QRS complex is actually present in the time period according to a determination that the reliability of the pulse wave is high.

13. A program configured to cause a computer to execute the physiological information processing method according to any one of claims 9 to 12.

## Patentansprüche

1. Gerät zum Verarbeiten physiologischer Informationen, umfassend:
einen oder mehrere Prozessoren; und
einen oder mehrere Speicher, die zum Speichern eines computerlesbaren Befehls ausgelegt sind,
wobei in dem Fall, dass der computerlesbare Befehl von dem Prozessor ausgeführt wird, das Gerät zum Verarbeiten physiologischer Informationen dazu eingerichtet ist:
Elektrokardiogrammdaten einer Testperson zu beziehen (S1);
einen ORS-Komplex aus den Elektrokardiogrammdaten zu erfassen (S2);
Pulswellendaten der Testperson zu beziehen (S11);
einen Stimulationsimpuls, der von einer von der Testperson getragenen Vorrichtung ausgegeben wird, zu bestimmen (S21);
zu bestimmen (S20, S21), ob der Stimulationsimpuls in einem Zeitabschnitt erfasst wird, in dem kein QRS-Komplex erfasst wird, wenn der Zeitabschnitt gleich oder länger als eine vorbestimmte Zeitdauer ist, und falls der QRS-Komplex aufgrund des Vorhandenseins des Stimulationsimpulses nicht erkannt wurde,
auf der Grundlage der Pulswellendaten zu bestimmen (S22), ob innerhalb einer vorgegebenen Zeit ab einem Erfassungszeitpunkt, zu dem der Stimulationsimpuls erfasst wird, eine Pulswelle vorhanden ist; und
entsprechend der Bestimmung, dass die Pulswelle innerhalb der vorgegebenen Zeit vorhanden ist, zu bestimmen (S24), dass der QRS-Komplex in dem Zeitabschnitt tatsächlich vorhanden ist.

2. Gerät zum Verarbeiten physiologischer Informationen nach Anspruch 1, wobei das Gerät zum Verarbeiten physiologischer Informationen dazu eingerichtet ist:
Daten bezüglich des QRS-Komplexes entsprechend der Bestimmung, dass die Pulswelle innerhalb der vorgegebenen Zeit vorhanden ist, zu aktualisieren; und
auf der Grundlage der Daten bezüglich des QRS-Komplexes zu ermitteln, ob ein Alarm, der eine Anomalie der Herzfunktion der Testperson anzeigt, akustisch oder visuell nach außen ausgegeben werden soll.

3. Gerät zum Verarbeiten physiologischer Informationen nach Anspruch 2,
bei dem die Daten bezüglich des QRS-Komplexes Informationen bezüglich eines Zeitintervalls zwischen benachbarten QRS-Komplexen und einer Zeitachse umfassen, und
das Gerät zum Verarbeiten physiologischer Informationen dazu eingerichtet ist, anhand eines Vergleichs zwischen dem Zeitintervall und einem vorgegebenen Schwellenwert zu ermitteln, ob der Alarm akustisch oder visuell nach außen ausgegeben werden soll.

4. Gerät zum Verarbeiten physiologischer Informationen nach einem der Ansprüche 1 bis 3,
wobei das Gerät zum Verarbeiten physiologischer Informationen dazu eingerichtet ist:
die Zuverlässigkeit der Pulswelle zu bestimmen (S23), wenn bestimmt wird, dass die Pulswelle innerhalb der vorgegebenen Zeit vorhanden ist; und
auf der Grundlage der Bestimmung, dass die Zuverlässigkeit der Pulswelle hoch ist, zu bestimmen, dass der QRS-Komplex in dem Zeitabschnitt tatsächlich vorhanden ist.

5. Gerät zum Verarbeiten physiologischer Informationen nach Anspruch 4,
wobei das Gerät zum Verarbeiten physiologischer Informationen dazu eingerichtet ist, die Zuverlässigkeit der Pulswelle durch Vergleich eines Merkmals der innerhalb der vorgegebenen Zeit vorhandenen Pulswelle mit einem Merkmal einer in dem Speicher gespeicherten Pulswelle zu ermitteln.

6. Gerät zum Verarbeiten physiologischer Informationen nach Anspruch 5,
wobei das Gerät zum Verarbeiten physiologischer Informationen dazu eingerichtet ist, die Zuverlässigkeit der Pulswelle zu ermitteln, indem es das Merkmal der innerhalb der vorgegebenen Zeit vorhandenen Pulswelle mit dem Merkmal der in dem Speicher gespeicherten Pulswelle vergleicht und indem es ermittelt, ob Rauschen auf der Pulswelle überlagert ist, die innerhalb der vorgegebenen Zeit vorhanden ist.

7. Gerät zum Verarbeiten physiologischer Informationen nach Anspruch 5 oder 6, bei dem das Merkmal der Pulswelle wenigstens eines der folgenden Merkmale umfasst:
ein Merkmal, das sich auf eine Amplitude der Pulswelle bezieht;
ein Merkmal, das sich auf die Kontraktionszeit der Pulswelle bezieht; und
ein Merkmal, das sich auf die Verzögerungszeit der Pulswelle bezieht, entsprechend einem Zeitintervall zwischen einem Erfassungszeitpunkt, zu dem die Pulswelle erfasst wird, und einem Erfassungszeitpunkt, zu dem der der Pulswelle zugeordnete QRS-Komplex oder der Stimulationsimpuls erfasst wird.

8. Gerät zum Verarbeiten physiologischer Informationen nach Anspruch 2 oder 3,
wobei das Gerät zum Verarbeiten physiologischer Informationen dazu eingerichtet ist, entsprechend der Bestimmung, dass die Pulswelle innerhalb der vorgegebenen Zeit vorhanden ist, die Daten bezüglich des QRS-Komplexes zu aktualisieren, indem sie einen Erfassungszeitpunkt, zu dem der Stimulationsimpuls erfasst wird, als Erfassungszeitpunkt speichert, zu dem der QRS-Komplex erfasst wird.

9. Verfahren zum Verarbeiten physiologischer Informationen, das von einem Computer ausgeführt wird, wobei das Verfahren umfasst:
Beziehen (S1) von Elektrokardiogrammdaten einer Testperson;
Erfassen (S2) eines QRS-Komplexes aus den Elektrokardiogrammdaten;
Beziehen (S11) von Pulswellendaten der Testperson;
Erfassen (S21) eines Stimulationsimpulses, der von einem in den Körper der Testperson implantierten Gerät ausgegeben wird;
Bestimmen (S20, S21), ob der Stimulationsimpuls in einem Zeitabschnitt erfasst wird, in dem kein QRS-Komplex erfasst wird, wenn dieser Zeitabschnitt gleich oder länger als eine vorgegebene Zeitbreite ist;
falls der QRS-Komplex aufgrund des Vorhandenseins des Stimulationsimpulses nicht erfasst wurde, Bestimmen (S22) auf der Grundlage der Pulswellendaten, ob innerhalb einer vorgegebenen Zeit ab einem Erfassungszeitpunkt, zu dem der Stimulationsimpuls erfasst wird, eine Pulswelle vorhanden ist; und
Bestimmen (S24) entsprechend der Bestimmung, dass die Pulswelle innerhalb der vorgegebenen Zeit vorhanden ist, dass der QRS-Komplex in dem Zeitabschnitt tatsächlich vorhanden ist.

10. Verfahren zum Verarbeiten physiologischer Informationen nach Anspruch 9, weiterhin umfassend:
Aktualisieren von Daten, die sich auf den QRS-Komplex beziehen, entsprechend der Bestimmung, dass die Pulswelle innerhalb der vorgegebenen Zeit vorhanden ist; und
Bestimmen, ob ein Alarm akustisch oder visuell nach außen ausgegeben werden soll, basierend auf den Daten, die sich auf den QRS-Komplex beziehen.

11. Verfahren zum Verarbeiten physiologischer Informationen nach Anspruch 10,
bei dem die Daten bezüglich des QRS-Komplexes Informationen bezüglich eines Zeitintervalls zwischen benachbarten QRS-Komplexen und einer Zeitachse umfassen, und
das Verfahren weiterhin das Bestimmen umfasst, ob der Alarm nach außen akustisch oder visuell ausgegeben werden soll, entsprechend einem Vergleich zwischen dem Zeitintervall und einem vorbestimmten Schwellenwert.

12. Verfahren zum Verarbeiten physiologischer Informationen nach einem der Ansprüche 9 bis 11, weiterhin umfassend:
Bestimmen (S23) der Zuverlässigkeit der Pulswelle, wenn bestimmt wird, dass die Pulswelle innerhalb der vorgegebenen Zeit vorhanden ist, und
das Verfahren ferner umfasst zu bestimmen, dass der QRS-Komplex in dem Zeitabschnitt tatsächlich vorhanden ist, basierend auf der Bestimmung, dass die Zuverlässigkeit der Pulswelle hoch ist.

13. Programm, das dazu eingerichtet ist, einen Computer zu veranlassen, das Verfahren zur Verarbeitung physiologischer Informationen gemäß einem der Ansprüche 9 bis 12 auszuführen.

## Revendications

1. Appareil de traitement d'informations physiologiques comprenant :
un ou plusieurs processeurs ; et
une ou plusieurs mémoires configurées pour stocker une instruction lisible par ordinateur,
dans lequel, dans un cas où l'instruction lisible par ordinateur est exécutée par le processeur, l'appareil de traitement d'informations physiologiques est configuré pour :
acquérir (S1) des données d'électrocardiogramme d'un sujet ;
détecter (S2) un complexe QRS à partir des données d'électrocardiogramme ;
acquérir (S11) des données d'onde de pouls du sujet ;
détecter (S21) une sortie d'impulsion de stimulation provenant d'un appareil porté par le sujet ;
déterminer (S20, S21) si l'impulsion de stimulation est détectée dans une période de temps dans laquelle un complexe QRS n'est pas détecté lorsque la période de temps est égale ou supérieure à une largeur de temps prédéterminée et si le complexe QRS n'a pas été détecté en raison de la présence de l'impulsion de stimulation,
déterminer (S22), sur la base des données d'onde de pouls, si une onde de pouls est présente dans un temps prédéterminé à partir d'un point temporel de détection au niveau duquel l'impulsion de stimulation est détectée ; et
déterminer (S24), selon une détermination que l'onde de pouls est présente dans le temps prédéterminé, que le complexe QRS est effectivement présent dans la période de temps.

2. Appareil de traitement d'informations physiologiques selon la revendication 1, dans lequel l'appareil de traitement d'informations physiologiques est configuré pour :
mettre à jour des données relatives au complexe QRS selon la détermination que l'onde de pouls est présente dans le temps prédéterminé ; et
déterminer s'il faut délivrer en sortie une alarme de manière sonore ou visuelle indiquant une anomalie d'une fonction cardiaque du sujet vers l'extérieur, sur la base des données relatives au complexe QRS.

3. Appareil de traitement d'informations physiologiques selon la revendication 2,
dans lequel les données relatives au complexe QRS comportent des informations relatives à un intervalle de temps entre des complexes QRS adjacents sur une ligne temporelle, et
dans lequel l'appareil de traitement d'informations physiologiques est configuré pour déterminer s'il faut délivrer en sortie l'alarme de manière sonore ou visuelle à l'extérieur conformément à une comparaison entre l'intervalle de temps et une valeur seuil prédéterminée.

4. Appareil de traitement d'informations physiologiques selon l'une quelconque des revendications 1 à 3,
dans lequel l'appareil de traitement d'informations physiologiques est configuré pour :
déterminer (S23) la fiabilité de l'onde de pouls lorsque l'onde de pouls est déterminée comme étant présente dans le temps prédéterminé ; et
déterminer que le complexe QRS est réellement présent dans la période de temps selon une détermination que la fiabilité de l'onde de pouls est élevée.

5. Appareil de traitement d'informations physiologiques selon la revendication 4, dans lequel l'appareil de traitement d'informations physiologiques est configuré pour déterminer la fiabilité de l'onde de pouls en comparant une caractéristique de l'onde de pouls présente dans le temps prédéterminé avec une caractéristique d'une onde de pouls stockée dans la mémoire.

6. Appareil de traitement d'informations physiologiques selon la revendication 5, dans lequel l'appareil de traitement d'informations physiologiques est configuré pour déterminer la fiabilité de l'onde de pouls en comparant une caractéristique de l'onde de pouls présente dans le temps prédéterminé avec la caractéristique d'une onde de pouls stockée dans la mémoire, et en déterminant si un bruit est superposé sur l'onde de pouls présente dans le temps prédéterminé.

7. Appareil de traitement d'informations physiologiques selon la revendication 5 ou 6, dans lequel la caractéristique de l'onde de pouls comporte au moins l'une parmi :
une caractéristique relative à une amplitude de l'onde de pouls ;
une caractéristique relative à un temps de contraction de l'onde de pouls ; et
une caractéristique relative à un temps de retard de l'onde de pouls correspondant à un intervalle de temps entre un point temporel de détection au niveau duquel l'onde de pouls est détectée et un point temporel de détection au niveau duquel le complexe QRS associé à l'onde de pouls ou à l'impulsion de stimulation est détecté.

8. Appareil de traitement d'informations physiologiques selon la revendication 2 ou 3, dans lequel l'appareil de traitement d'informations physiologiques est configuré pour mettre à jour, selon la détermination que l'onde de pouls est présente dans le temps prédéterminé, les données relatives au complexe QRS en stockant un point temporel de détection au niveau duquel l'impulsion de stimulation est détectée comme point temporel de détection au niveau duquel le complexe QRS est détecté.

9. Procédé de traitement d'informations physiologiques exécuté par un ordinateur, le procédé comprenant les étapes consistant à :
acquérir (S1) des données d'électrocardiogramme d'un sujet ;
détecter (S2) un complexe QRS à partir des données d'électrocardiogramme ;
acquérir (S11) des données d'onde de pouls du sujet ;
détecter (S21) une sortie d'impulsion de stimulation provenant d'un appareil implanté dans un corps du sujet ;
déterminer (S20, S21) si l'impulsion de stimulation est détectée dans une période de temps dans laquelle un complexe QRS n'est pas détecté lorsque la période de temps est égale ou supérieure à une largeur de temps prédéterminée ;
si le complexe QRS n'a pas été détecté en raison de la présence de l'impulsion de stimulation, déterminer (S22), sur la base des données d'onde de pouls, si une onde de pouls est présente dans un temps prédéterminé à partir d'un point temporel de détection au niveau duquel l'impulsion de stimulation est détectée ; et
déterminer (S24), selon une détermination que l'onde de pouls est présente dans le temps prédéterminé, que le complexe QRS est effectivement présent dans la période de temps.

10. Procédé de traitement d'informations physiologiques selon la revendication 9, comprenant en outre :
la mise à jour de données relatives au complexe QRS selon la détermination que l'onde de pouls est présente dans le temps prédéterminé ; et
le fait de déterminer s'il faut délivrer en sortie une alarme de manière sonore ou visuelle à l'extérieur sur la base des données relatives au complexe QRS.

11. Procédé de traitement d'informations physiologiques selon la revendication 10,
dans lequel les données relatives au complexe QRS comportent des informations relatives à un intervalle de temps entre des complexes QRS adjacents sur une ligne temporelle, et
dans lequel le procédé comporte en outre le fait de déterminer s'il faut délivrer en sortie l'alarme de manière sonore ou visuelle à l'extérieur conformément à une comparaison entre l'intervalle de temps et une valeur seuil prédéterminée.

12. Procédé de traitement d'informations physiologiques selon l'une quelconque des revendications 9 à 11, comprenant également :
la détermination (S23) de la fiabilité de l'onde de pouls lorsque l'onde de pouls est déterminée comme étant présente dans le temps prédéterminé, et
dans lequel le procédé comporte en outre la détermination que le complexe QRS est effectivement présent dans la période de temps selon une détermination que la fiabilité de l'onde de pouls est élevée.

13. Programme configuré pour amener un ordinateur à exécuter le procédé de traitement d'informations physiologiques selon l'une quelconque des revendications 9 à 12.
